# DEMANDE DE BREVET EUROPEEN

(11) **EP 0 695 540 A1**
(43) Date de publication de la demande: **07.02.1996**
(21) Numéro de dépôt: 95420227.1
(22) Date de dépôt: 02.08.1995
(51) Int. Cl.: A61F 2/36

(54) **Prothèse de hanche**

(30) Priorité: 02.08.1994 FR 9409825
(71) Demandeur: MERCK BIOMATERIAL FRANCE, F-01800 Charnoz (FR); Hulin, Paul Henri, F-89000 Auxerre (FR); Commarmond, Jacques, F-45200 Montargis (FR); Durand, Jean-Claude, F-58000 Nevers (FR)
(72) Inventeur: Hulin, Paul Henri, F-89000 Auxerre (FR); Commarmond, Jacques, F-45200 Montargis (FR); Durand, Jean-Claude, F-58000 Nevers (FR); Collomb, Jean, F-26800 Portes les Valence (FR)
(74) Mandataire: Laurent, Michel

(57) **Abrégé**

Cette prothèse de hanche comprend une tige fémorale (1) destinée à être enfouie dans le fémur à prothéser, et un col prothétique (2), dont l'extrémité (3) reçoit une tête destinée à coopérer avec un noyau de frottement destiné à être reçu dans la cavité cotyloïdienne de l'articulation considérée.

Le profil général de la tige fémorale (1) présente une antétorsion se développant sensiblement de la zone métaphysodiaphysaire à la zone épiphysaire de la tige.

## Description

L'invention concerne un nouveau type de prothèse de hanche.

De manière connue, une prothèse de hanche est fondamentalement constituée d'une tige fémorale, destinée à être insérée dans le canal médulaire du fémur de l'articulation de la hanche considérée, et un col prothétique surmonté d'une tête, faisant ou non partie intégrante de la tige, et destinée à correspondre à son extrémité avec un noyau de frottement, généralement réalisé en polyéthylène et destiné à coopérer avec la cavité cotyloïdienne de ladite hanche au niveau de laquelle est préalablement inséré ou mis en place un cotyle de forme appropriée correspondante.

De plus en plus, on cherche à réaliser ces prothèses de la manière la plus anatomique possible, afin notamment de permettre une meilleure adaptabilité de la prothèse au fémur considéré, et partant de favoriser l'intégration osseuse. En définitive, on vise à augmenter la résistance mécanique de l'ensemble, mais également à diminuer au maximum les contraintes parasites, principalement inhérentes au porte à faux, et autres pressions exercées compte-tenu du caractère non-anatomique des prothèses à ce jour disponibles, engendrant des douleurs pour le patient et des remaniements osseux indésirables.

Dans l'esprit de cette démarche, il avait été proposé, notamment dans le document EP-A-0 485 311, une prothèse à antéversion modulable, dans laquelle le col prothétique était rapporté au niveau de la portion proximale de la tige fémorale, après mise en place de celle-ci, l'angle d'antéversion étant choisi en per-opératoire par le praticien lors de l'intervention, afin d'adapter au mieux la prothèse à la morphologie et à l'anatomie du patient.

Si certes, ce principe d'antéversion modulable a permis d'accomplir des progrès importants, notamment au niveau de l'articulation, en diminuant notamment les risques de luxation etc..., se posait toujours le problème du caractère anatomique de la tige proprement dite, qui n'était toujours pas satisfait par ce type de prothèse, puisqu'il n'était pas tenu compte de l'antéflexion et de la courbure sagitale du fémur, ni du caractère hélitorsé de la métaphyse proximale fémorale.

De fait, on continue à observer outre des descellements de la tige fémorale au niveau du fémur, nonobstant la qualité des ciments utilisés ou des revêtements de surface, égalements des douleurs post-opératoires, compte tenu de l'appui de la tige fémorale contre la corticale interne du fémur.

Avant de s'affranchir de certains de ces inconvénients, on a proposé, par exemple dans les documents EP-A-0 128 036 et WO 91/18560, de conférer à la zone proximale des prothèses un profil tel, que la partie supérieure de cette zone est vrillée, notamment dans le sens de l'antéversion du col prothétique. De la sorte, il devient ainsi possible d'adapter le profil général des prothèses à l'antéversion naturelle du fémur, et ainsi de limiter les douleurs engendrées par la mise en place de telles prothèses.

Cependant, si certes ces nouveaux profils semblent mieux adaptés à l'anatomie du fémur, ils demeurent insatisfaisants au moins du point de vue de la fixation de la prothèse au niveau de cette articulation.

L'invention a pour objet de résoudre ces problèmes. Elle concerne une prothèse de hanche comprenant une tige fémorale destinée à être enfouie dans le fémur à prothéser, et un col prothétique dont l'extrémité reçoit un noyau de frottement destiné à son tour à être reçu dans la cavité cotyloïdienne de l'articulation considérée, dans laquelle le profil général de la tige fémorale présente une antétorsion se développant sensiblement de la zone métaphysodiaphysaire à la zone épiphysaire de la tige.

En d'autres termes, l'invention consiste à incurver la tige, notamment en zone proximale, de telle sorte à conférer à celle-ci un profil hélitorsé par rapport à l'axe longitudinal passant par le centre de l'extrémité inférieure de la zone distale de ladite tige, le pas de ce profil hélicoïdale étant largement supérieur à la hauteur totale de la tige, ladite zone proximale subissant en outre une rotation partielle sur elle-même, se développant sensiblement de la zone métaphysodiaphysaire à la zone épiphysaire de la tige, également en hélice. L'invention consiste donc à conférer au profil de la zone proximale de la tige une double modification, d'une part, par rapport à l'axe fictif de la tige proprement dite, passant de manière connue par le centre de la zone distale, et d'autre part par rapport à l'axe théorique de la prothèse, défini comme étant l'axe passant par le centre de symétrie de chacune des sections réalisées perpendiculairement à cet axe théorique, cet axe présentant donc une courbure commençant sensiblement au niveau de la zone métaphysodiaphysaire.

De manière plus imagée, le profil général de la tige subit une modification de sa morphologie, à l'instar d'une hélice dont le pas est très grand, puisque l'angle d'antétorsion moyen est de 10 °. De la sorte, on peut ainsi définir une tige fémorale droite et une tige fémorale gauche, en fonction du lieu d'implantation de la prothèse considérée.

Selon une caractéristique avantageuse de l'invention, le col prothétique peut être distinct de la tige fémorale et ainsi être rapporté au niveau de la zone proximale de la tige, de telle sorte à combiner l'antétorsion du fémur à l'antéversion modulable du col, pour ainsi optimiser le caractère anatomique de la prothèse en résultant, et ainsi favoriser l'implantation de celle-ci au niveau de l'articulation prothesée.

La manière dont l'invention peut être réalisée et les avantages qui en découlent ressortiront mieux de l'exemple de réalisation qui suit, à l'appui des figures annexées, dans lesquelles :
- la figure 1 est une vue schématique de face de la prothèse selon l'invention ;
- la figure 2 est une vue dans le plan sagital de ladite prothèse ;
- la figure 3 est une réalisation schématique représentant une superposition de sections transversales de la tige, notamment permettant de montrer l'incurvation ou le cintrage de la zone proximale de la tige et l'hélitorsion de celle-ci, conformément à l'invention ;
- les figures 4 et 5 sont des vues dans le plan sagital de la tige de l'invention, respectivement sans et avec col prothétique ;
- la figure 6 est une représentation schématique en perspective partielle du col prothétique rapportable sur la tige fémorale ;
- les figures 7 et 8 sont des coupes sagitales schématiques de la tige fémorale après et avant mise en place du col prothétique rapporté.

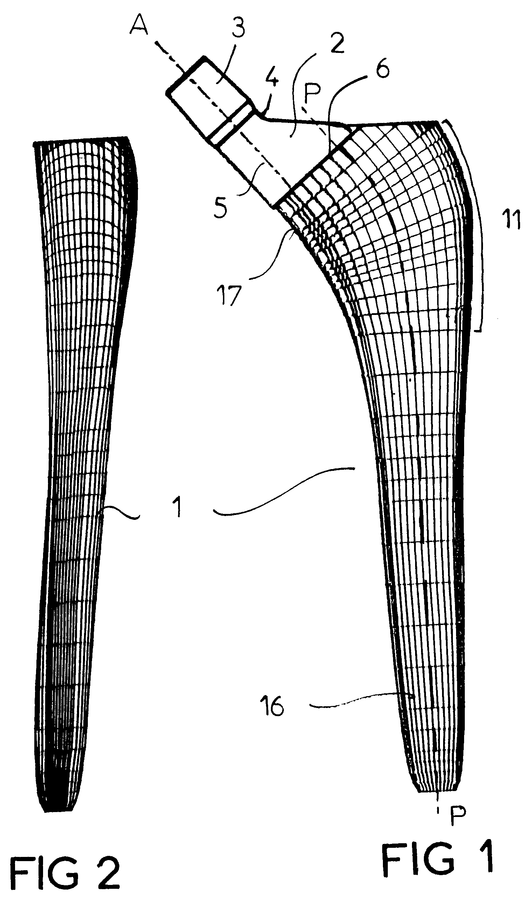

On a représenté schématiquement sur la figure 1 une prothèse de hanche conforme à l'invention et plus spécifiquement la tige fémorale portant la référence générale (1). Cette tige fémorale (1) se prolonge à son extrémité proximale par un col prothétique désigné par la référence (2). De manière connue, le col prothétique (2), destiné à dépasser du fémur à prothéser, comprend tout d'abord un support de tête cylindrique ou légèrement tronconique (3), destinée à recevoir par emmanchement une sphère non représentée sur laquelle s'appuie la cupule cotyloïdienne ou noyau de frottement. Cette sphère est avantageusement réalisée en acier inoxydable ou en céramique.

Le support de tête (3) est raccordé par un col (4) à une base (5), dont l'embase (6) est sensiblement perpendiculaire à l'axe A du col prothétique lorsque l'angle d'antéversion du col prothétique est de zéro degré.

Selon les formes de réalisation de l'invention, le col prothétique (2) fait partie intégrante de la tige fémorale (1) (tige monobloc) ou est indépendant de la tige et est rapporté sur celle-ci après mise en place de la tige fémorale au niveau du fémur à prothéser.

Selon une caractéristique fondamentale de l'invention, le profil de la tige fémorale présente une forme particulière notamment au niveau de la partie proximale (11).

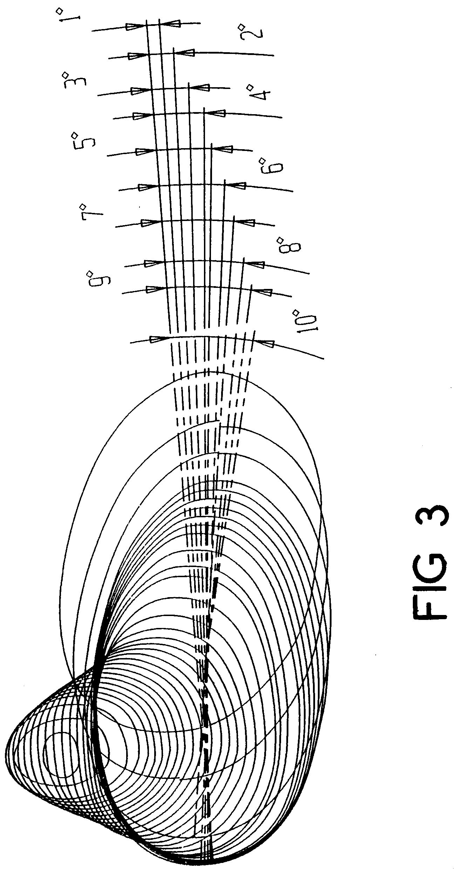

Plus spécifiquement, le profil de la tige fémorale présente une antétorsion entre la zone diaphysaire, et plus précisément métaphysodiaphysaire, c'est à dire sensiblement centrale, jusqu'à la zone supérieure proximale de la tige. Cette antétorsion correspond d'une part à une incurvation de la tige au niveau d'au moins l'une de ses faces latérales, et d'autre part, à une torsion hélicoïdale dans sa partie proximale supérieure, en direction de la droite ou de la gauche, selon le fémur à prothéser droit ou gauche, ainsi que l'on peut mieux l'observer sur la figure 3. Bien que le terme face latérale puisse paraître impropre, attendu que la section transversale de la tige ne correspond pas à un parallélogramme, mais davantage à une ellipse, on doit entendre par face latérale l'un des côtés de la tige parallèle au grand axe de la dite ellipse correspondant sensiblement à l'intersection de n'importe lequel des coupes transversales de la tige en zone proximale avec un plan perpendiculaire à l'axe théorique P - P de ladite tige.

Autrement dit, la portion proximale (11) de la tige s'écarte du plan de symétrie longitudinale de sa portion distale (16). Cet écart latéral se double d'une légère hélitorsion en direction de la face interne (17) de la tige, cette dernière constituant un référentiel fixe à partir duquel s'effectue les homothéties de rapport déterminé pour aboutir à une gamme complète de tiges. En d'autre termes, cette face interne (17) conserve ses caractéristiques constantes, afin de restituer le cintre obturateur du fémur. Ce profil particulier de la tige est mieux perceptible sur la figure 3, dans laquelle on a représenté des sections successives de la tige en partant de l'extrémité de la portion distale (16) jusqu'à l'extrémité de la portion proximale (11), cette représentation étant effectuée selon une vue réalisée suivant l'axe X - X de la figure 1. On observe ainsi, sur cette figure 3, tout d'abord une incurvation de la tige vers le bas de ladite figure, puis une torsion vers la face interne de la tige, cette torsion étant profilée sensiblement en hélice, de pas est très grand, l'angle d'antétorsion moyen étant de 10 °.

Selon une caractéristique fondamentale de l'invention, cette antétorsion s'accompagne d'une rotation ou d'un pivotement de la tige sur elle-même au niveau de sa portion proximale (11), ainsi que l'on peut également l'obersver sur la figure 3, dans laquelle, on a représenté la variation angulaire du grand axe de l'ellipse constitutif des sections de la dite portion réalisées perpendiculairement à l'axe théorique P - P, par rapport à l'axe desdites sections avant commencement de ce mouvement de pivot. On observe ainsi, que la dernière section présente un angle de 10° par rapport à l'axe d'origine.

Le profil résultant d'une part de l'antétorsion de la tige et de ce mouvement de vrille de la portion proximale, confère à la prothèse ainsi constituée, outre une meilleure capacité d'adaptation au profil anatomique du fémur à prothéser, réduisant de fait les douleurs post-opératoires, également une fixation de la prothèse au sein dudit fémur largement optimisée, éliminant de manière très significative les risques de descellements.

Pour tenir compte de l'antéverion moyenne du fémur des individus, la valeur angulaire de cette antétorsion, mesurée entre le plan de symétrie distale de la tige et l'axe A du col prothétique lorsque celui-ci est en place sur la tige avec une valeur d'antéversion nulle, est voisine de 10 °. Du fait de cette caractéristique, on obtient un meilleur remplissage du canal médulaire du fémur à prothéser par la tige, notamment au niveau de sa partie métaphysaire. Corollairement, on observe une meilleure distribution des contraintes au niveau de la paroi corticale du fémur, et partant une diminution des douleurs éventuellement ressenties par le patient, et surtout une meilleure intégration osseuse, avec élimination des remaniements osseux parasites.

De la sorte, cette meilleure adaptation anatomique de la tige aboutit, outre à sa mise en place mieux adaptée, également à une fixation plus précise, mieux harmonisée et plus ferme compte-tenu de la plus grande surface de contact entre la tige elle-même et la corticale interne du fémur. De plus, si la tige est fixée sans ciment, on observe une optimisation de la fixation secondaire inhérente à une meilleure repousse osseuse.

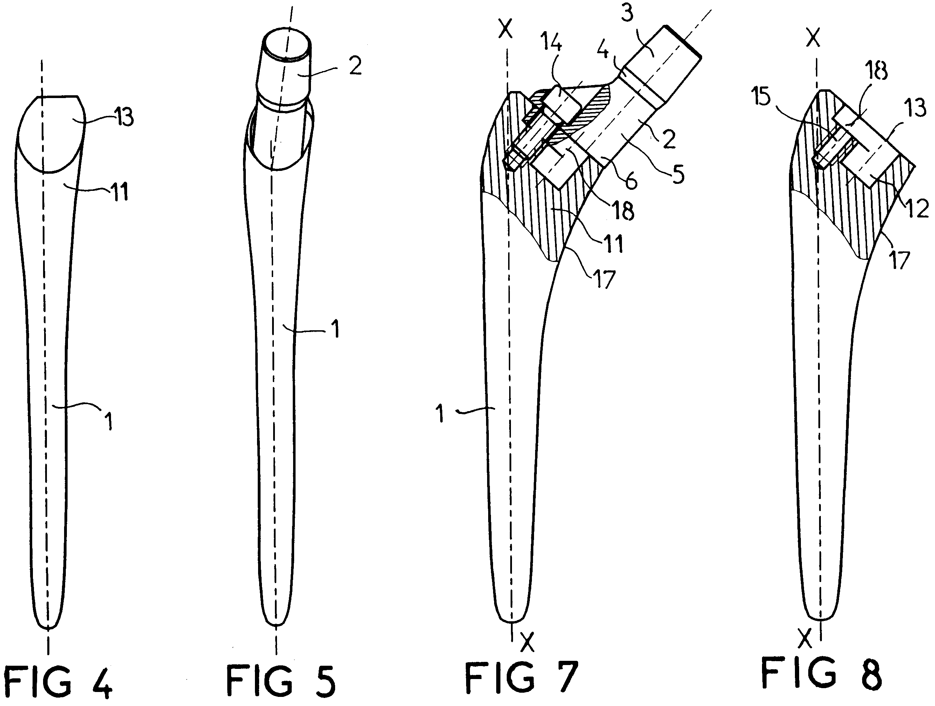

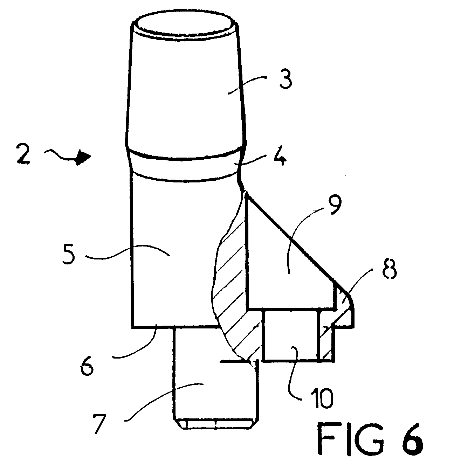

L'antétorsion de la tige peut avantageusement s'accompagner d'une antéversion en agissant au niveau du col prothétique (2), lorsque celui-ci est rapporté au niveau de la portion proximale de la tige, ainsi que cela a été décrit notamment en liaison avec les figures 4 à 8.

Ainsi donc, le col prothétique (2) susceptible d'être rapporté, comporte une portée cylindrique ou légèrement tronconique d'emmanchement (7), orthogonale par rapport à l'embase (6), l'extrémité de cette portée cylindrique étant avantageusement légèrement biseautée.

Cette portée (7) est destinée à coopérer avec un logement cylindrique (12), ménagé au niveau supérieur de la portion proximale (11) de la tige, et s'étendant à partir de l'embase (13) de ladite portion proximale.

Parallèlement, la base (5) du col prothétique (2) présente une lumière (9) se prolongeant par un orifice traversant (10), pour permettre le passage d'une vis de fixation (14), destinée à coopérer avec un orifice taraudé (15) ménagé à côté du logement cylindrique ou tronconique selon le cas (12) de la portion proximale (11) de la tige, et orienté parallèlement à celui-ci.

Enfin, l'embase (13) de la portion proximale de la tige présente une lumière sensiblement ovale (18), destinée à coopérer avec une forme complémentaire ménagée au niveau de l'embase (6) du col prothétique (2). Ainsi, cette coopération forme-lumière permet d'obtenir une indexation immédiate en bonne position lors de la mise en place du col sur la tige, et permet en outre d'aboutir à un renfort du rôle mécanique en flexion de la portée d'emmanchement ou cône (12), et en rotation de la vis (14).

Ainsi, après mise en place de la tige au niveau du fémur à prothéser, tige présentant de fait une antétorsion de 10 °, le praticien en cours d'opération procède à la sélection de l'angle d'antéversion en choisissant le col prothétique le plus approprié à l'anatomie du patient.

De sorte que l'angle total entre le plan de symétrie longitudinal de la zone distale (16) de la tige et le plan vertical passant par notamment l'axe A du col prothétique, peut varier entre 10° et 30° (en antéversion ou en rétroversion) en fonction de l'anatomie du patient et du choix de l'angle d'antéversion du col prothétique par le praticien. On notera que les deux embases de raccordement respectivement (6) et (13), parallèles, sont disposées à la limite et légèrement en dessus de la zone d'enfouissement de la tige au sein du fémur, permettant avantageusement d'éviter d'entailler le grand trochanter, et de fragiliser le fémur, puisque l'ostéotomie du col fémoral est réalisée à 45° par rapport à l'axe fémoral, en partant de la fosse digitale jusqu'à aboutir à environ 15 mm au dessus du petit trochanter.

De la sorte, l'invention présente les avantages déjà connus des tiges à antéversion modulable et à savoir notamment la facilité de pose du chirurgien compte-tenu de l'absence de col, et la possibilité d'obtenir déjà une première adaptation anatomique, auxquels se rajoutent également les avantages liés à l'antétorsion déjà mentionnés, et qui résident principalement dans une augmentation de l'adaptation anatomique de la tige au fémur, et notamment à l'antéflexion de celui-ci, et partant à optimiser la soliditié de sa mise en place.

En outre, l'utilisation d'un col prothétique rapporté permet en cas de ré-intervention, notamment pour changer les pièces dites d'usure, telles que cupule polyéthylène, etc..., de n'enlever seulement que le col, diminunant la complexité de l'intervention, et simplifiant le "geste" opératoire.

Enfin, du fait du principe de modularité de l'antétorsion globale de la prothèse, le praticien dispose d'une véritable panoplie de choix, rendant superflu dans la majorité des cas, le recours à une prothèse sur mesure, et partant diminunant de façon drastique les coûts, notamment d'intervention.

## Revendications

**1/** Prothèse de hanche comprenant une tige fémorale (1) destinée à être enfouie dans le fémur à prothéser, et un col prothétique (2), dont l'extrémité (3) reçoit une tête destinée à coopérer avec un noyau de frottement destiné à être reçu dans la cavité cotyloïdienne de l'articulation considérée, ***caractérisée*** en ce que le profil général de la tige fémorale (1) présente une antétorsion se développant sensiblement de la zone métaphysodiaphysaire à la zone épiphysaire de la tige.

**2/** Prothèse de hanche selon la revendication 1, ***caractérisée*** en ce que la tige (1) est incurvée notamment au niveau de sa zone proximale (11), selon un profil héli-torsé par rapport à l'axe longitudinal X - X passant par le centre de l'extrémité inférieure de la zone distale (16) de ladite tige et en direction de la face interne (17) de la tige, ladite tige présentant au niveau de sa zone proximale (11) une rotation partielle sur elle-même se développant sensiblement de la zone métaphysodiaphysaire à la zone épiphysaire de la tige.

**3/** Prothèse de hanche selon l'une des revendications 1 et 2, ***caractérisée*** en ce que la zone proximale (11) de la tige fémorale (1) est incurvée par rapport au plan de symétrie longitudinale de la portion distale (16) de ladite tige, et en ce que ladite zone proximale (11) adopte un profil hélicoïdale dont le pas est largement supérieur à la hauteur totale de la tige.

**4/** Prothèse de hanche selon l'une des revendications 1 à 3, ***caractérisée*** en ce que l'angle moyen d'antétorsion, mesuré entre le plan de symétrie longitudinale de la portion distale (16) de la tige et l'axe A du col prothétique (2) lorsque celui-ci est en place sur la tige avec une valeur d'antéversion nulle est voisin de 10 degrés.

**5/** Prothèse de hanche selon l'une des revendications 1 à 4, ***caractérisée*** en ce que le col prothétique (2) est indépendant de la tige fémorale (1) et est rapporté au niveau de l'embase (13) de la zone proximale (11) de la tige, ledit col définissant une angle d'antéversion qui lui est propre, de telle sorte à combiner l'antétorsion de la tige à l'antéversion modulable du col.

**6/** Prothèse de hanche selon la revendication 5, ***caractérisée*** en ce que le col prothétique présente une embase plane (6), parallèle à l'embase (13) de la portion proximale (11) de la tige une fois en place, embase (6) à partir de laquelle émerge une saillie de forme sensiblement ovale, d'où est issue une portée cylindrique ou légèrement tronconique d'emmanchement (7), orthogonale à ladite embase (6), et destinée à coopérer avec un logement correspondant (12), ménagé au sein de ladite portion proximale (11), la fixation réversible du col prothétique (2) sur la tige (1) étant assurée au moyen de la coopération vis (14) - écrou intégré (15), la vis (14) étant reçue dans un logement (9) prévu à cet effet au sein de la base (5) du col (2) et prenant appui sur un épaulement traversant (10) colinéaire avec le logement et parallèle à la portée (7), l'écrou intégré (15) étant également parallèle au logement (13), et ménagé au sein de la portion proximale (11) de la tige, la saillie ovale de l'embase (6) du col prothétique étant elle-même destinée à coopérer avec une lumière de même dimension ménagée au niveau de l'embase (13) de la portion proximale (11) de la tige, afin notamment de permettre l'indexation directe et immédiate du col lors de sa mise en place sur la tige.

**7/** Prothèse de hanche comprenant une tige fémorale (1) destinée à être enfouie dans le fémur à prothéser, et un col prothétique (2), dont l'extrémité (3) reçoit une tête destinée à coopérer avec un noyau de frottement destiné à être reçu dans la cavité cotyloïdienne de l'articulation considérée, ***caractérisée*** en ce que le profil général de la tige fémorale (1) présente une antétorsion se développant sensiblement de la zone métaphysodiaphysaire à la zone épiphysaire de la tige, cette antétorsion se décomposant en une torsion hélicoïdale de la tige par rapport à l'axe fictif X - X de la tige passant par le centre de la portion distale (16) de ladite tige, le pas de ladite hélice étant largement supérieur à la hauteur totale de la tige, et en une rotation du profil de la zone proximale (11) de la tige se traduisant par une rotation du grand axe de l'ellipse définie par l'intersection de la tige avec l'axe théorique P - P de la tige.

**8/** Prothèse de hanche selon la revendication 7, ***caractérisée*** en ce que la rotation du profil de la portion proximale (11) de la tige est d'au plus 10 degrés au niveau de son extrémité supérieure.
